# EUROPEAN PATENT SPECIFICATION

(11) **EP 2 837 698 B1**
(45) Date of publication and mention of the grant of the patent: **13.09.2017**
(21) Application number: 13724628.6
(22) Date of filing: 12.04.2013
(51) Int. Cl.: C12R 1/85, C12G 1/00

(54) **SACCHAROMYCES CEREVISIAE STRAIN**
SACCHAROMYCES CEREVISIAE STAMM
SOUCHE DE SACCHAROMYCES CEREVISIAE

(30) Priority: 13.04.2012 PT 10625213
(43) Date of publication of application: 18.02.2015
(73) Proprietor: Yeastwine - Wine Solutions, Lda., 4730-575 Soutelo - Vila Verde (PT); Universidade do Minho, 4704-553 Braga (PT)
(72) Inventor: GONÇALVES VIEIRA, Maria Eugénia, P-4710-428 Braga (PT); SCHULLER, Dorit Elisabeth, 4715-338 Braga (PT); AMORIM CASAL, Margarida Paula Pedra, 4715-123 Braga (PT); MORAIS ARAÚJO, Isabel Maria, 4715-037 Braga (PT)
(74) Representative: Silvestre de Almeida Ferreira, Luís Humberto
(86) International application number: PCT/IB2013/052934
(87) International publication number: WO 2013/153540

(56) References cited:
- MARIN BEROVIC ET AL: "Influence of Temperature and Carbon Dioxide on Fermentation of Cabernet Sauvignon Must", FOOD TECHNOL. BIOTECHNOL., vol. 41, no. 4, 31 December 2003 (2003-12-31), pages 353-359, XP55068634, ISSN: 1330-9862
- ANDORRA IMMA ET AL: "Effect of pure and mixed cultures of the main wine yeast species on grape must fermentations", EUROPEAN FOOD RESEARCH AND TECHNOLOGY, vol. 231, no. 2, June 2010 (2010-06), pages 215-224, XP002699691, ISSN: 1438-2377
- RODRIGUEZ-PORRATA B ET AL: "Vitality enhancement of the rehydrated active dry wine yeast", INTERNATIONAL JOURNAL OF FOOD MICROBIOLOGY, ELSEVIER BV, NL, vol. 126, no. 1-2, 15 August 2008 (2008-08-15), pages 116-122, XP023315605, ISSN: 0168-1605, DOI: 10.1016/J.IJFOODMICRO.2008.05.016 [retrieved on 2008-05-22]
- MARIAN REDÃ N ET AL: "Effect of growth temperature on yeast lipid composition and alcoholic fermentation at low temperature", EUROPEAN FOOD RESEARCH AND TECHNOLOGY ; ZEITSCHRIFT FÜR LEBENSMITTELUNTERSUCHUNG UND -FORSCHUNG A, SPRINGER, BERLIN, DE, vol. 232, no. 3, 9 January 2011 (2011-01-09), pages 517-527, XP019884980, ISSN: 1438-2385, DOI: 10.1007/S00217-010-1415-3
- VALERO E ET AL: "Dissemination and survival of commercial wine yeast in the vineyard: A large-scale, three-years study", FEMS YEAST RESEARCH, WILEY-BLACKWELL PUBLISHING LTD, GB, NL, vol. 5, no. 10, 1 July 2005 (2005-07-01), pages 959-969, XP027680515, ISSN: 1567-1356 [retrieved on 2005-07-01]

## Description

### Technical field of the invention

The present invention relates to a new *Saccharomyces cerevisiae* strain characterized by its deposit on the March 2012 in the "Colección Española de Cultivos Tipo" (CECT) in Valencia - Depositary of the Budapest Treaty - under number CECT 13073, herein designated by AV12, which can be preferably applied for initiating and conducting food alcoholic fermentations, particularly fruit / cereal musts, such as for wine, beer or cider productions.

The strain herein described allows to the production of persistent, full bodied and smooth wines, with pronounced fruity aroma.

### Background of the Invention

Over the centuries yeasts have been used worldwide in the production of foods and beverages, but was in the last decades, accompanying the development of genetic characterization of microorganisms, that great strides have been made in the selection of yeast species and strains for food industry, allowing the improvement of the products quality and / or the efficiency of the industrial process.

Wine is the result of an alcoholic fermentation, a microbial process which is performed by yeasts that convert the must into wine.
In spontaneous fermentations this conversion is performed by the yeasts present on the grapes surface or inside the cellar, but the fermentation efficiency and the wine quality is often unpredictable.

The addition of selected yeast cultures to initiate and conduct the alcoholic fermentation, also called yeast starter or active dry yeast (ADY) is currently a widespread winemaking practice.

The inoculation with the same ADY ensures, besides the control of the fermentation process, the wine characteristics uniformity over the years of production. There are about 200 commercially available ADY and there is demand for wine yeast strains with new oenological properties or with the ability to improve them.

In Portugal, despite being a country with a long winemaking tradition, only three *S. cerevisiae* strains, isolated from Vinhos Verdes, Bairrada and Dão regions, are marketed. Considering that in Portugal there are 13 wine-producing regions and nearly 250 different grapevine cultivars, there is the possibility of isolating different *S. cerevisiae* strains adapted to several conditions.

Traditionally yeast selection criteria were based on its fermentative power, fermentation kinetics appropriate to different temperatures, low acetic acid production and sulfur dioxide (SO₂ resistance. With the recognition that yeast has a role that extends beyond the fermentation, and that the metabolites produced by certain yeast strains have a sensory impact in the wine, other criteria were also considered (Pretorius, 2000).
The quality of a wine is also results from a complex balance of all odorous compounds sensed during sensory analysis, which includes aroma evaluation. The aroma results from a biochemical and technological sequence and is decisively influenced by the fermentation process. The volatiles metabolites generated during sugars fermentation by *S. cerevisiae* yeasts are mostly esters, higher alcohols, volatile fatty acids, carbonaceous compounds, and volatile sulfur compound. These compounds contribute to the sensory profile of the wine, and their accumulation in the final product depends on the yeast strain, the must composition and fermentation conditions. Additionally, volatile compounds can also be released through the action of enzymes produced by the yeast on non-volatile compounds, aromas precursors, which are present in grape skin. Examples of such compounds are the monoterpenes and C13-norisoprenoids, which are released from glycosidic precursors, and long-chain polyfunctional thiols, which are derived from S-cystenyl conjugates (Moreno-Arribas et Polo, 2009).

The higher alcohols are volatile compounds produced in greater quantities during fermentation and, according to Versini and Rapp (1996), they add a desirable aromatic complexity to the wine, when present in concentrations below 300 mg/L, but in higher concentrations they may have a negative impact. The concentrations of each higher alcohols acting positively or negatively on wine aroma, tend to depend on aroma intensity and wine style. On one hand, they help to solubilize compounds with limited solubility, such as the ones with high molecular weight. The solubilization of these compounds may contribute to the wine aroma profile. On the other hand, in very high concentrations they can impart "vinous" aroma, and in wines with lower concentrations they help to highlight other varietal aromas (Suarez-Lepe et al, 2011). There are many factors influencing the higher alcohols formation, including the yeast species and strain and the amount of inoculum, initial sugar content, fermentation temperature, pH and must composition, assimilable nitrogen, aeration, level of solids, grape cultivar and the time that must spends in contact with grape skins (Carrau et al, 2010). The main polyols formed during fermentation are glycerol and 2,3-butanediol (Suárez-Lepe et Morata, 2011). Glycerol is a major by-product of the glucose conversion during alcoholic fermentation, carried out by yeast, and is produced mainly in the beginning of fermentation (Ribéreau-Gayon, 2006). The amount of glycerol present in a wine dependents on many conditions. In addition to the strain and the amount of inoculum used, also factors such as aeration, fermentation temperature, nitrogen must composition, sugars, sulfites and pH, grape cultivar and grapes infection by *Botrytis cinerea* (during which glycerol is produced) may influence the final glycerol concentration (Radler et Schutz, 1982; Scanes et al, 1998). Typically, under controlled fermentation conditions, the glycerol concentrations are higher in red wines than in white ones. The values normally present in wines, found in the literature, vary according to the authors, ranging from 1-15 g/L (Scanes et al, 1998) to 8.5 g/L (Ribéreau-Gayon, 2006) being the average value of 7 g/L (Scanes et al, 1998). The taste threshold of perception for glycerol is 5.2 g/L in the wine, while the increase in viscosity is perceptible only from 25 g/L (Scanes et al, 1998).

Glycerol has a direct impact on the flavor characteristics of the wine due to its non-volatile nature, but has a slightly sweet taste and contributes to the wine smoothness, consistency and body (Pretorius, 2000; Scanes et al, 1998). Studies have been carried out on genetically modified yeast in order to obtain higher amounts of glycerol (Cambon et al, 2006; Cordier et al, 2007). There is even the document US6140108 which describes a way for wine yeast improvement through the obtainment of hybrid strains able to increase the glycerol production. However, the studies on genetically modified yeasts conducted so far, show that the increase in glycerol production is accompanied by an increase of other products which have a negative impact on the wine quality, such as acetic acid and acetaldehyde (Scanes et al, 1998; Cambon et al, 2006).

The 2,3-butanediol increases the softness of the wine, integrating better the polyphenol fraction. The concentration of this compound in wine depends on the fermentation conditions and on the yeast strain used. The wine structure and softness can be increased using yeast strains that produce higher amounts of 2,3-butanediol (Morata et Lepe-Suarez, 2011).

The volatile thiols are important aroma components from wines produced from different grape cultivars, in particular Sauvignon Blanc, but also from Scheurebe, Gewürztraminer, Riesling, Cabernet Sauvignon, Merlot, among others, and can have significant impact on the quality of the wine, corresponding to the consumer preferences (Swiegers et al, 2007a). These compounds are virtually absent in grapes and musts, occurring only in the course of fermentation due to the yeasts action in the cleavage of non-odorous precursors (Tominaga et al. 1995). The major volatile thiols are 4-mercapto-4-methylpentan-2-one (4MMP), 3-mercaptohexanol-1-ol (3MH) and 3-mercaptohexil acetate (3AMH), which in optimal concentrations confer aromas such boxwood, passion fruit, grapefruit, gooseberry, guava and lychee (Swiegers et al, 2007a), and at excessive concentrations can confer aromas of cat sweat and urine(Dubourdieu et al, 2000). The thresholds of perception of these compounds are 0.8 ng/L (4MMP), 60 ng/L (3MH) and 4 ng/L (3AMH) (Tominaga et al. 1998a).

The interest for the contribution of volatile thiols in wine increased considerably during the last 20 years. Much research has been done on their synthesis in wine production in order to manage the quality of wines in which these compounds are present. As an example, the document US2011/00451440A1 describes two yeast strains of the species *Pichia kluyveri,* which act synergistically with some commercial yeast strains to produce high concentrations of volatile thiols.

Rodriguez-Porrata et al, 2008 determined the best yeast rehydration conditions.

Berovic, M. 2003 discloses the influence of variation of the process temperature and the fluxes of additional inlet gaseous carbon dioxide in *Saccharomyces bayanus* fermentation.

Redón, M. 2011 discloses that the loss of viability of wine yeast strains due to low temperature fermentations may be overcome by increasing their stress tolerance and adaptability.

Andorra, I. 2010 discloses that mixed inoculation of non-*Saccharomyces* yeasts and *S. cerevisiae* is of interest for the wine industry.

### Summary of the Invention

With the new *S. cerevisiae* strain characterized by the deposit CECT 13073, hereinafter referred to as AV12, described in the present invention, it is intended to broaden options of wine, cider or beer producers, providing them a tool that does not require additional costs or changes in the production process, allowing to obtain a new product. The wines obtained from the fermentation with this new strain will match the consumer preferences, since those wines acquire fruity characteristics (in particular tropical fruit in white wines), have a lingering taste and are full-bodied and soft. These sensory characteristics are largely due the synergy of several aromatic compounds, such as esters, higher alcohols, terpenes, norisoprenoids and volatile thiols, as well as the significant amounts of glycerol and 2,3-butanediol.

This invention aims to describe a new *S. cerevisiae* characterized by the deposit CECT 13073, which can be used in production of fermented food (including beverages, preferably the ones obtained by must fermentation, such as wine, beer or cider.

The *S. cerevisiae* strain mentioned above has the following characteristics:
- tolerance to ethanol concentrations between 14 - 16% (v/v);
- resistant to SO₂ (100 mg/L);
- Glycerol production higher than 7 g/L, preferably in the red wines and in half of the white wines, from the tested cultivars;
- increased production of 2,3-butanediol, 30% higher compared to wine produced under the same winemaking conditions with the QA23^{®} yeast - wine strain marketed by Proenol company;
- increased production of aromatic compounds such as volatile thiols, higher alcohols, esters of fatty fixed, monoterpenes and norisoprenoids, in comparison with wine produced under the same winemaking conditions with the yeast QA23^{®} - wine strain marketed by Proenol company.

Thus, the strain described in this invention combines a set of functional features that in its entirety, and achieving a synergistic effect, are distinct from the characteristics presented by the QA23^{®} strain, as well as the general knowledge of the field.

It is a further object of this invention the transformation of the above described strain (AV12) by metabolic engineering, for example, enhancing its ethanol tolerance by increasing the number of copies of the ethanol tolerant genes RCN1 and RSA3, or through evolutionary engineering, for example, the adaptation process of the yeast strain to increasing ethanol concentrations.

Another object of the present invention is the winemaking process using the *S. cerevisiae* strain characterized by the deposit CECT 13073.

In a preferred embodiment, the vinification process comprises the following steps of:
- adding to the must, preferably from white or red grapes, the strain described above in appropriate amounts;
- conducting the fermentation at a temperature between 16-28 °C;
- an efficient must remounting potentiates the aromas produced by the yeast.

In a more preferred embodiment, grapes of the following cultivars can be used:
- white grape cultivars - Batoca, Loureiro, Alvarinho, Sauvignon Blanc, Chardonnay, Gewürztraminer, Riesling or their mixtures;
- red grape cultivars - Merlot, Cabernet Sauvignon, Syrah, Touriga Nacional, Vinhao or their mixtures.

In another preferred embodiment of the winemaking process, already described, the fermentation temperature:
- in white grape musts can range between 16-20 °C, preferably 18-20 °C;
- in red grape musts can range between 25-28 °C.
In one embodiment, further preferably, the *S. cerevisiae* strain characterized by the deposit CECT 13073 may be used in its dried, lyophilized or paste forms.

### General description of the invention

This invention regards a novel *S. cerevisiae* characterized by the deposit CECT 13073, preferably used to initiate and conduct fermentations in musts, in order to ensure a controlled fermentation, without the development of malodors.
This new strain is suited to the general vinification conditions, being tolerant to ethanol (14%, v/v), resistant to SO₂ (100 mg/L) and a medium H₂S producer. Wines produced with this new strain show intense fruity aromas, in particular tropical fruit aromas in white wines. The taste persistence and a feeling of warmth and greasiness are other features of the wines produced with this new yeast. These sensory attributes are associated with the production of volatile thiols, aromatic esters, norisoprenoids, terpenes, acetates and higher alcohols, regarding the flavor and the production of glycerol and 2,3 - butanediol with regard to flavor.

### Detailed Description of the Invention

The globalization of the wine market has increased the international competition from winemaking industry, which currently needs to quickly respond to consumers demand for different wines styles.
This invention aims to describe a new *S. cerevisiae* strain characterized by the deposit CECT 13073 preferentially used to initiate and conduct fermentations of must, ensuring controlled fermentations and producing full-bodied, persistent and soft wines with fruity aromas. The use of new wine yeasts, as the one described in this invention, allows the producer to obtain, in a simple and fast way, unique wines due to the huge impact that this organism has in the wine sensory characteristics.

The *S. cerevisiae* strain characterized by the deposit CECT 13073 was isolated from spontaneous fermentations of grapes collected in various wine regions and the distinction of the *S. cerevisiae* strains was performed by interdelta sequence analysis (Embodiment 1.1).

A first oenological qualitative characterization showed that the strain is resistant to ethanol and SO₂ (14% v/v and 100 mg/L, respectively) and is a medium H₂S producer (Embodiment 1.2).

The microfermentations (100 mL) held in Loureiro/Avesso 2010 musts, showed that the fermentation profiles of the selected strain and of the commercial strain QA23^{®} were similar. This behavior was further confirmed during the vinification held in 50 L of Loureiro/Avesso 2010 must (Embodiment 1.3).

In addition to the oenological characteristics mentioned above, another feature that influenced the selection of this strain was the unexpected and intense aroma of tropical fruits, particularly passion fruit aroma, and the feeling of warmth and softness in mouth (Embodiment 1.2).

Sensory analysis, performed by a panel of 34 tasters, of the wines produced in parallel (in a volume of 50 L) of Loureiro/Avesso 2010, with the selected strain and the commercial strain, revealed that wine produced with the selected strain has a more intense fruity aroma, more persistent taste and conveys a greater sense of warmth (Embodiment 1.3).

The selected strain has the capacity to ferment different types of musts, namely both from white or red grape cultivars, as demonstrated in the harvest of 2011, in cellar context, with the cultivars Loureiro, Batoca, Alvarinho, Sauvignon Blanc, Chardonnay, Gewürztraminer, Riesling, Merlot, Cabernet Sauvignon, Syrah, Touriga Nacional and Vinhao (Embodiment 1.4).

Fermentations were performed at different temperatures with the selected strain, which allowed the verification of the good fermentation behavior in the range of 16 to 28 °C (Embodiment 1.4).

The main chemical and sensory characteristics of the wines obtained with the selected strain, do not change with increased fermentation scale, as was seen with Loureiro 2011 must (5.50 and 500 L) and with Loureiro/Avesso 2010 must (100 mL and 50 L) (Embodiments 1.3 and 1.4).

Fermentations were performed in parallel with the selected strain and the commercial strain QA23^{®} in musts of the cultivars Loureiro/Avesso and Batoca, and with the selected strain in a spontaneous fermentation of grapes from Vinhao cultivar. Comparing the wines obtained with each of both strains, it was found that in all cases the wines obtained with the selected strain had higher glycerol concentration values (Embodiments 1.3 and 1.4). It was further found that also for the compound 2,3-butanediol, concentrations 30% higher were obtained with the selected stain (Embodiments 1.4). The glycerol concentration values obtained in fermentations performed with the selected strain, of grapes from the different grape cultivars that were tested (white and red), were higher than the average value of 7 g/L. These significant glycerol concentrations are not associated with acetic acid concentrations above the threshold of perception (Embodiments 1.4). The compounds with the highest odor activity value (OAV) from the wines produced with the selected strain, showed descriptors of a fruity character, while most of the OAV compounds of the wines produced with the strain QA23^{®} show descriptors of a more floral character (Embodiments 1.4).

### Description of the Figures

For an easier understanding of the invention figures are attached, which represent preferred embodiments of the invention, however not intended to limit the scope of this invention.
**Figure 1****:** Graphic representation of the fermentation profiles obtained with *S. cerevisiae* strain characterized by the deposit CECT 13073, called AV12, and the commercial strain QA23^{®} in a volume of 50 L of Loureiro / Avesso 2010 must, at 18 °C.
**Figure 2****:** Graphic representation of the fermentation products (ethanol and glycerol), residual sugars (fructose) and organic acids (tartaric, malic, succinic and acetic) quantified by HPLC analysis of the wines obtained by fermentation in 50 L, and in the microfermentations of 100 mL of Loureiro / Avesso 2010 must with the *S. cerevisiae* strain, characterized by the deposit CECT 13073, called AV12 strain, and with the commercial strain QA23^{®}.
**Figure 3****:** Graphical representation of the sensory profile of the wines produced with the selected *S. cerevisiae* strain, characterized by the deposit CECT 13073, called AV12, and with the commercial strain QA23^{®}.
**Figure 4****:** Graphical representation of glycerol and acetic acid produced during fermentation, quantified by HPLC analysis of the wines from the red grape cultivars (Touriga Nacional, Syrah, Merlot, Cabernet Sauvignon and Vinhao), obtained by the fermentation of 50 L of must with the *S. cerevisiae* strain characterized by the deposit CECT 13073, called AV12, and with the indigenous yeast microflora in the Vinhao cultivar and from wines from the white grapes cultivars Alvarinho (50 L), Sauvignon Blanc (50 L), Chardonnay (5 L), Gewürztraminer (5 L) and Riesling (5 L).
**Figure 5****:** Graphical representation of the average concentrations of volatile compounds (by families of volatile compounds) present in the wines.

### Preferred Embodiment

### 1.1 Yeast Isolation

In one preferred embodiment, 66 grape samples were collected from Portuguese grapevine cultivars (Aragonês, Avesso, Baga, Castelao, Loureiro and Touriga Nacional) in 11 vineyards, belonging to the Portuguese wine regions of Vinhos Verdes, Dão, Douro, Bairrada, Ribatejo and Alentejo.
Spontaneous fermentations were carried out with the obtained musts (500 mL, 22 °C), which were monitored by daily weight loss determination (CO₂ release). In the final stage of the fermentation must aliquots was withdrawn and after plating in YPD medium (1% yeast extract, w/v, peptone 1%, w/v, 2% glucose, w/v and 2% agar, w/v), 30 colonies were isolated and stored (glycerol, 30% v/v; -80 °C) for later DNA extraction, making a total of 1500 individual yeast isolates.

The *S. cerevisiae* strains delimitation was performed by interdelta sequence analysis. Delta sequences (300 bp) flank the Ty2 and TY1 retrotransposons are dispersed in the *S. cerevisiae* genome and are very polymorphic markers, since their number and location exhibit intraspecific variability (Schuller et al., 2004, Legras et Karst, 2003). Thus, after DNA isolation (Lopez et al, 2001; Schuller et al, 2004) the amplification of DNA segments between delta sequences was carried out using the amplification conditions and the primers δ2 and δ12 previously described (Schuller et al. 2004, Legras Karst et al, 2003). Amplicons were separated in agarose gels (1.5%, w / v) and the analysis of the obtained profiles allowed to the differentiation of 64 *S. cerevisiae* strains.

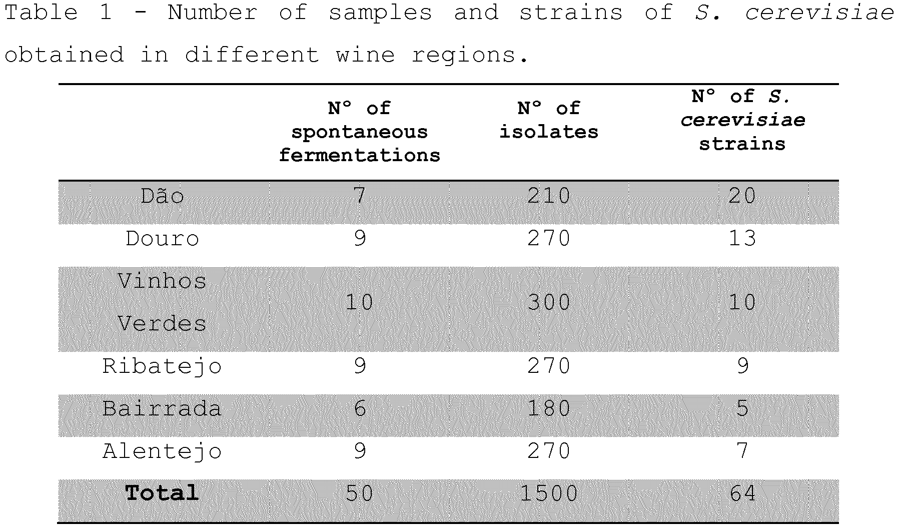

### 1.2 Selecting the yeast

An initial qualitative enological characterization of the identified strains was carried out in order to estimate the production of hydrogen sulfide (H₂S) and evaluate the resistance to sulfur dioxide (SO₂) and ethanol. To that end, the 64 *S. cerevisiae* strains and a commercial strain (QA23^{®}) grew overnight in YPD liquid medium (30°C, 200 rpm), and cell suspensions were adjusted to the same optical density (at 550 nm) of 1.0. Each strain was tested in Biggy agar medium (Fluka) for the H₂S production, and in Malt Extract agar medium (Fluka) with different ethanol and SO₂ concentrations (12, 14, 16 and 18% v/v and 50, 75 and 100 mg/L, respectively). Microfermentations (18 °C) were also performed for each of the strains and for the commercial strain QA23^{®} in 100 mL of Loureiro/Avesso 2010 must, that was sterilized by filtration and inoculated in order to obtain a concentration of 1x10⁶ cells/mL. The experiment allowed to i) a preliminary sensory evaluation of the wines, ii) access the fermentation profile of each of the strains, iii) and to quantify certain fermentation products (ethanol and glycerol), residual sugars (fructose) and organic acids (tartaric, malic, acetic and succinic acids) of each wine obtained. Integrating all data, a promising *S. cerevisiae* strain characterized by the deposit CECT 13073 was selected. In addition to its good fermentation profile, the selected stain revealed an unexpected and intense aroma of tropical fruits, particularly the passion fruit aroma, and surprised by the feeling of warmth and softness, unusual in the final stage of fermentation of wines from those grape cultivars.

The results of the qualitative evaluation concerning H₂S production, SO₂ resistance, and resistance to ethanol of the *S. cerevisiae* characterized by the deposit CECT 13073, compared to the commercial strain QA23^{®} (Table 2) indicate that both strains are resistant to ethanol and SO₂ (14% v/v and 100 mg/L, respectively) and are medium H₂S producers. The fermentation profile of the selected strain was similar to the one from the commercial strain and the preliminary sensory evaluation revealed tropical fruit aromas, namely an intense passion fruit aroma.

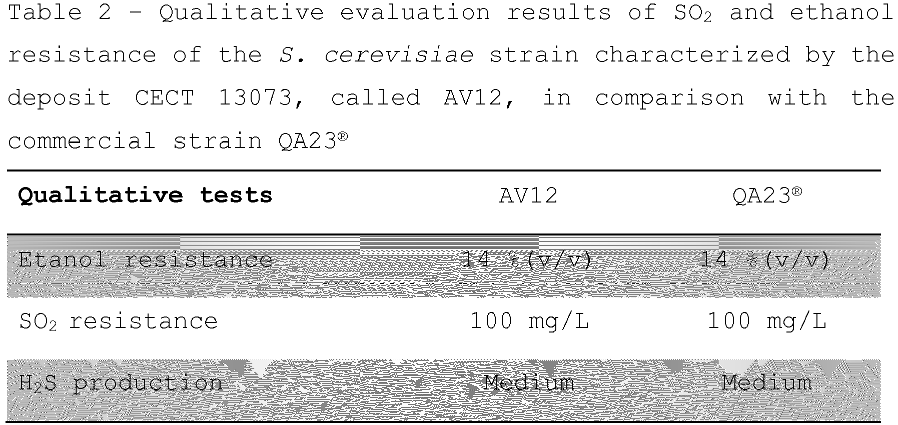

### 1.3 Vinifications

Fermentations were performed in parallel with the selected *S. cerevisiae* strain characterized by the deposit CECT 13073, and the commercial yeast QA23^{®}, in a larger volume (50 L) of Loureiro/Avesso 2010 must, at 18°C, in order to confirm the fermentative behavior observed in smaller scale. The yeast cultures addition was performed in the form of "fresh culture" ie, yeasts were not lyophilized.Thus, was initially prepared a pre-inoculum in 50 mL of YPD medium (1% yeast extract, w/v, peptone 1%, w/v and 2% glucose, w/v) from cultures stored at - 80° C, which grew for 24 hours (30 °C, 200 rpm). The inoculum was performed in 1% (v/v) of the total fermentation volume for 24-48 h in YPD medium (30 °C, 200 rpm), to achieve a concentration of approximately 1x10⁸ cells/mL. Thus, by inoculating that suspension on the total fermentation volume, an initial cell concentration of about 1x10⁶ cells/mL was obtained.

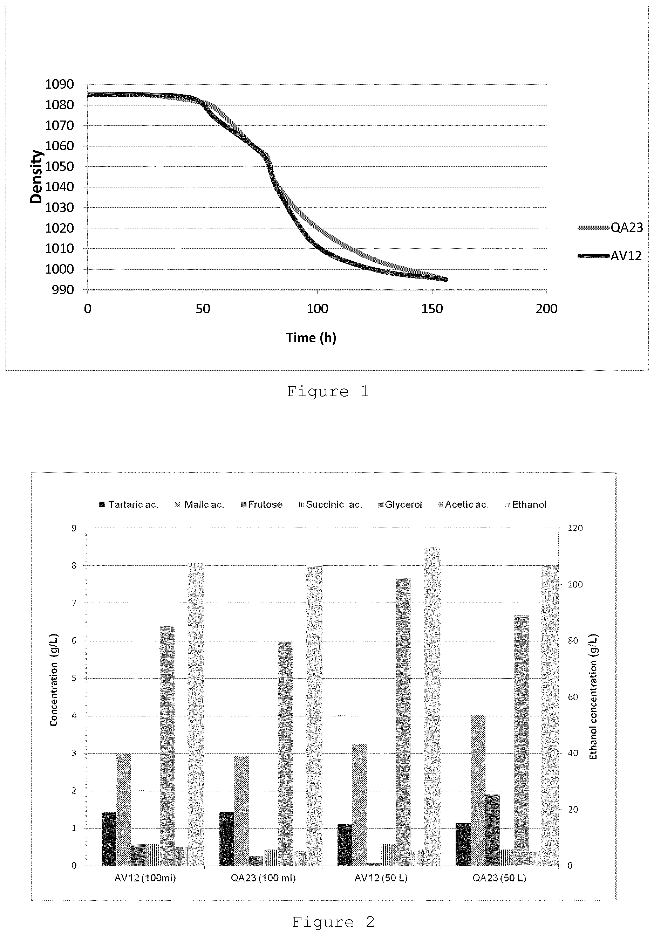

In the fermentation conditions described above, the profile of the fermentation conducted with *S. cerevisiae* strain characterized by the deposit CECT 13073 was similar to the fermentation profile of the commercial strain in the beginning of the fermentation, but was more effective than the commercial strain in the final stages (Figure 1).

The concentrations of fermentation products (ethanol and glycerol), residual sugars (fructose) and organic acids (tartaric, malic, acetic and succinic) were quantified by HPLC analysis of both wines obtained by the fermentations of Loureiro/Avesso 2010 must, with each of both strains in comparison. The wine obtained by fermentation with the *S. cerevisiae* strain characterized by the deposit CECT 13073 showed higher ethanol and glycerol concentrations and low fructose concentration, which is in accordance with the best performance observed at the end of fermentation of this strain (Figure 2). Results obtained in 50 L fermentations were comparable to those observed in microfermentations that were previously performed (Figure 2).

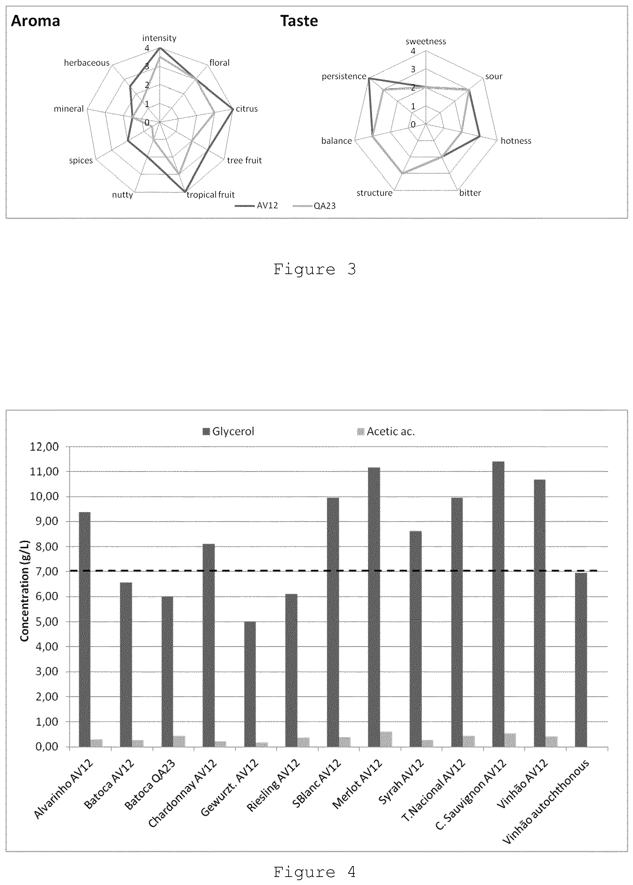

The produced wines were evaluated by a panel of 34 trained and untrained tasters, in order to meet the common consumer preferences. The sensory analysis evaluated the aroma (intensity, floral, tropical fruit, citrus, tree fruits, nuts, spices, mineral and vegetable) and flavor (sweetness, acidity, heat, bitterness, structure, balance and persistence) on a scale from zero (not sensed) to five (very strong). The sensory profiles of both wines are shown in Figure 3.

Sensory analysis revealed that the wine produced with the *S. cerevisiae* strain characterized by the deposit CECT 13073 has a more intense fruity aroma, has a more persistent taste and conveys a greater sense of warmth. These sensory features were supported by chromatographic analysis, as ethanol and glycerol contribute to the body and heat of the wine (Figure 2), and higher alcohols, esters, organic acids and acetates contribute to the fruity notes (Figure 6).

### 1.4 Vinification with several grape cultivars

In 2011, comparison tests between both strains were repeated in 5 L of Batoca must, a grape cultivar little aromatic.

Fermentations were also carried out in different must volumes (in triplicate 5 L, 50 L, 500 L) of Loureiro from 2011, with the *S. cerevisiae* strain characterized by the deposit CECT 13073, being the fermentations of 5 and 50 L performed at a temperature of 18-20 °C and the fermentation of 500 L at 16 °C. The fermentation behavior of the *S. cerevisiae* strain characterized by the deposit CECT 13073, was further tested in musts of other grape cultivars, in particular white ones (at 18-20 °C) as Alvarinho (50 L), Sauvignon Blanc (50 L), Chardonnay (5 L), Gewürztraminer (5 L), Riesling (5 L), and red grape cultivars (25-28 °C) as Merlot (50 L), Cabernet Sauvignon (50 L), Syrah (50 L) and Touriga Nacional (50 L). An assay was further performed comparing the fermentation of grape musts from the cultivar Vinhao (50 L) using the selected strain characterized by the deposit CECT 13073 with a spontaneous fermentation (adding no yeast) of the same must in the same conditions. The concentrations of the fermentation products, residual sugars and organic acids, were quantified by HPLC analysis.

The results obtained in the different volumes of Loureiro were similar, showing the results reproducibility in larger scale fermentations. It was observed that all wines from the red grape cultivars and from three white grape cultivars (Alvarinho, Chardonnay and Sauvignon Blanc) produced with the selected *S. cerevisiae* strain characterized by the deposit CECT 13073 had glycerol concentrations above the mean value reported in the literature. In wines produced with grapes from Batoca cultivar, although the glycerol concentrations were lower than 7 g/L, it was found that the wine produced with the *S. cerevisiae* strain characterized by the deposit CECT 13073 had a higher concentration than the wine produced with the strain QA23^{®}. Also in wines from Vinhao cultivar, the wine produced with the *S. cerevisiae* strain characterized by the deposit CECT 13073 showed a glycerol concentration higher than the wine produced in the spontaneous fermentation (adding no yeast). Note that, despite high levels of glycerol, only the wine from Merlot cultivar showed an acetic acid value close to its threshold of perception.

The values of 2,3-butanediol were obtained by the method described by Peinado (2004), slightly modified, and it was observed that the values obtained with the *S. cerevisiae* strain characterized by the deposit CECT 13073 were 30% higher in comparison to the values obtained with the commercial strain QA23^{®}.

The preliminary sensory analysis of the red wines revealed that the *S. cerevisiae* strain characterized by the deposit CECT 13073 accentuates the aromatic characteristics of the cultivar and gives persistence and smoothness to the wine. The wines were analyzed by the method of Oliveira et. al. (2009) and there were identified and quantified a total of 65 compounds, among which three C6 compounds, 12 higher alcohols, six ethyl esters of fatty acids, seven ethyl esters of fixed acids, five acetates, six monoterpene alcohols, seven oxides and monoterpene diols, two C13 norisoprenoids, five volatile phenols, eight volatile fatty acids, one carbonyl compound and also 2-metiltetrahidrotiofeno-3-one, 2-metil tetrahidrotiofeno-3-one, pantolactone and N-(2-phenylethyl)-acetamide. All compounds were identified by the comparison of their mass spectra and retention times with the ones from reference compounds.

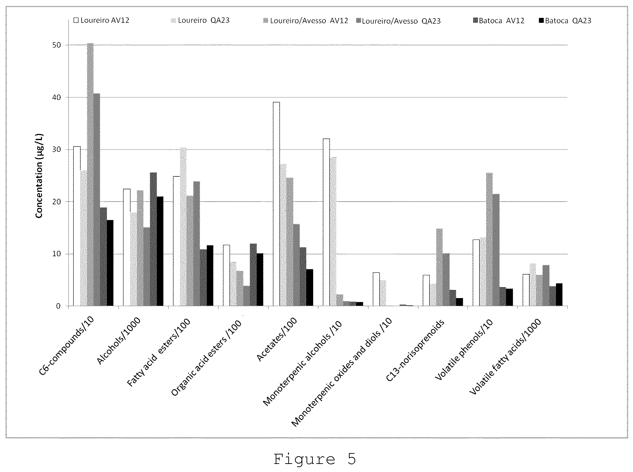

From the ten families of compounds that were identified and quantified, the higher alcohols were quantitatively the largest group in all wines. Concerning the other families of compounds, the wines produced with the strain characterized by the deposit CECT 13073 have a higher amount than those produced with the strain QA23^{®}, excluding esters of fatty acids and volatile fatty acids (Figure 5). In order to evaluate the influence of the studied compounds on the overall wine aroma, the odor activity value (OAV) was calculated, dividing the concentration of each compound by its threshold of perception. Only those compounds with OAV greater than one, impact on the flavor (Guth, 1997). At least 25% of the tested compounds contribute to the aroma of the wines (were not found in the literature values of the threshold of perception for all compounds). For all grape cultivars, the wines produced with the *S. cerevisiae* strain AV12 have a higher number of compounds with OAV greater than one. The compounds with the highest OAV present in the wines produced with the *S. cerevisiae* strain AV12, showed descriptors with a fruity character, while most of the compounds of the wines produced with strain QA23^{®} showed descriptors of a more floral character. The highest OAV were obtained for esters, compounds which are responsible for fruity aromas of the wines. All wines produced with the selected strain showed values higher than one, for all analyzed esters. These wines also obtained twice the OAV for higher alcohols, 2 and 3-methyl-1-butanol (descriptor for banana) and for isoamyl acetate (descriptor for banana and apple) in comparison to the wines produced with the commercial strain QA23^{®}.
The volatile thiols (4MMP, 3AMH and 3MH) were quantified by the method of extraction / derivatization - GC / MS in the wines obtained by fermentation of Loureiro must with the *S. cerevisiae* strain characterized by the deposit CECT 13073 and with the QA23^{®} strain. Both wines had values above the threshold of perception for the compounds 4MMP and 3AMH, and the wine produced with the *S. cerevisiae* strain characterized by the deposit CECT 13073 showed higher concentrations for those three compounds (10% higher for 4MMP and 3MH).

The *S. cerevisiae* strain characterized by the deposit CECT 13073 suits the usual winemaking conditions, since it was tested on different red and white grape cultivars, fermented at different temperatures (16, 18, 18-20, 25-28 °C) and the observed fermentation profile, as expected also for other commercial yeasts, took on average 3-4 days to complete the fermentation of red grape musts and 6 to 10 days of white grape musts. Industrial scale (500 L) assays were performed, in which the *S. cerevisiae* strain characterized by the deposit CECT 13073 and the commercial strain were under the same fermentation conditions. Fermentation was successfully performed within the expected time frame. The wines obtained through fermentations with the selected yeast strain have characteristics in common such as the smoothness, the persistence and the feeling of warmth that they convey. The wines from the tested white grape cultivars showed stronger fruity aroma, including tropical fruit aromas; in the wines from the tested red grape varieties, the characteristics of the cultivars are accentuated.

### Bibliografia

- Anderson, M. J., Barker, S. L., Boone, C., & Measday, V. (2012). Identification of RCN1 and RSA3 as ethanol tolerant genes in Saccharomyces cerevisiae using a high copy barcoded library. FEMS Yeast Research, 12, 48-60. doi:10.1111/j.1567-1364.2011.00762.x.
- Andorrá, I., Berradre, M., Rozès, N., Mas, A., Guillamón, J. M., & Esteve-Zarzoso, B. (2010). Effect of pure and mixed cultures of the main wine yeast species on grape must fermentations. European Food Research and Technology, 231(2), 215-224.
- Berovic, M., Mavri, J., Wondra, M, Kosmerl, T., & Bavcar, D., (2003). Influence of temperature and carbon dioxide on fermentation of Cabernet Sauvignon must. Food Technol. Biotechnol. 41(4), 353-359.
- Cambon, B., Monteil, V., Remize, F., Camarasa, C., & Dequin, S. (2006). Effects of GPD1 overexpression in Saccharomyces cerevisiae commercial wine yeast strains lacking ALD6 genes. Applied and environmental microbiology, 72(7), 4688-4694.
- Carrau, F. M., Medina, K., Farina, L., Boido, E., Henschke, P. a, & Dellacassa, E. (2008). Production of fermentation aroma compounds by Saccharomyces cerevisiae wine yeasts: effects of yeast assimilable nitrogen on two model strains. FEMS yeast research, 8, 1196-1207.
- Cordier, H., Mendes, F., Vasconcelos, I., & François, J. M. (2007). A metabolic and genomic study of engineered Saccharomyces cerevisiae strains for high glycerol production. Metabolic engineering, 9, 364-378.
- Darriet P, Tominga T, Lavigne V, Boidron J, Dubourdieu D. (1995). Identification of a powerful aromatic compound of Vitis vinifera L. var. Sauvignon wines: 4-mercapto-4-methylpentan- 2-one. Flavour Fragr J 10: 385-392.
- Dinh, T. N., Nagahisa, K., Hirasawa, T., Furusawa, C., & Shimizu, H. (2008). Adaptation of Saccharomyces cerevisiae cells to high ethanol concentration and changes in fatty acid composition of membrane and cell size. PloS one, 3(7), e2623.
- Dubourdieu DT, Tominaga I, Masneuf C, et al. (2000). The role of yeasts in grape flavor development during fermentation: the example of Sauvignon blanc. American Society of Enology and Viticulture, 50th Annual Meeting, Seattle, WA, 19-23 June 2000; 196-203.
- Goddard, M. R., Gardner, R. C., & Anfang, N. (2011) - US20110045140A1.
- Guth, H. (1997). Identification of character impact odorants of different white wine varieties, J. Agric. Food Chem. 45, 3027-3032.
- Legras, J.-L., & Karst, F. (2003). Optimisation of interdelta analysis for Saccharomyces cerevisiae strain characterisation. FEMS Microbiology Letters, 221(2), 249-255.
- Lopez, Victoria, Amparo Querol, Daniel Ramon, and M Teresa Fernandez-Espinar. (2001). A simplified procedure to analyse mitochondrial DNA from industrial yeasts. Int. J. Food Microbiol. 68: 75-81.
- Mortimer, R. K., Prior, B., & Baccari, C. (2000). US006140108A.
- Oliveira, J.M., Genisheva, Z., Lima, L., Vilanova, M., (2009). Easy and accurate methodology to quantify volatile compounds in fermented beverages. In: VIth Symposium in Vino Analytica Scientia. Angers, France.
- Peinado, R., Moreno, J., Muñoz, D., Medina, M., & Moreno, J. (2004). Gas Chromatographic Quantification of Major Volatile Compounds and Polyols in Wine by Direct Injection. Journal of agricultural and food chemistry, 52, 6389-6393.
- Pretorius, I. S. (2000). Tailoring wine yeast for the new millennium: novel approaches to the ancient art of winemaking. Yeast, 16, 675-729.
- Radler, F., & Schutz, H. (1982). Glycerol Production of varius strains of Saccharomyces. Am. J. Enol. Vitic., 33(1), 36-40.
- Rapp, A.; Versini, G; 1996: Flüchtige phenolische Verbindungen in Wein. Deutsche Lebensmittel-Rundschau 92, 42-48.
- Redón, M., Guillamón, J. M., Mas, A., & Rozès, N. (2011). Effect of growth temperature on yeast lipid composition and alcoholic fermentation at low temperature. European Food Research and Technology, 322 (3), 517-527.
- Ribereau-Gayon, P., Dubourdieu, D., Donéche, B., & Lonvaud, A. (2006). Handbook of Enology - Volume 1 The Microbiology of Wine and Vinifications. (John Wiley & Sons, Ed.)Microbiology (2nd ed., Vol. 1, pp. 0-470).
- Rodriguez-Porrata, B., Novo, M., Guillamón, J., Rozès, N., Mas, A., & Cordero Otero, R. (2008). Vitality enhancement of the rehydrated active dry wine yeast. International Journal of Food Microbiology, 126(1-2), 116-122.
- Scanes, K. T., Hohmann, S., & Prior, B. A. (1998). Glycerol production by the yeast saccharomyces cerevisiae and its relevance to wine - a review. S. Afr. J. Enol. Vitic., 19(1), 17-24.
- Schuller, D., Valero, E., Dequin, S., & Casal, M. (2004). Survey of molecular methods for the typing of wine yeast strains. FEMS microbiology letters, 231(1), 19-26.
- Suárez-Lepe, J. A., & Morata, A., (2011) New trends in yeast selection for winemaking, Trends in Food Science & Technology, XX, 1-12;
- Swiegers, J. H., & Pretorius, I. S. (2007b). Modulation of volatile sulfur compounds by wine yeast. Applied microbiology and biotechnology, 74, 954-960.
- Swiegers, J. H., Bartowsky, E. J., Henschke, P. A., & Pretorius, I. S. (2005). Yeast and bac- terial modulation of wine aroma and flavour. In R. J. Blair, M. E. Francis, & I. S. Pre- torius (Eds.), Advances in wine science (pp. 159-187). Glen Osmond, Australia: The Australian Wine Research Institute.
- Swiegers, J. H., Capone, D. L., Pardon, K. H., Elsey, G. M., Sefton, M. A., Francis, I. L., & Pretorius, I. S. (2007a). Engineering volatile thiol release in Saccharomyces cerevisiae for improved wine aroma. Yeast, 24, 561-574.
- Tominaga T, Masneuf I, Dubourdieu D. (1995). A S-cysteine conjugate, precursor of aroma of white sauvignon. J Int Sci Vigne Vin 29: 227-232.
- Tominaga, T., Gachons, C. P., & Dubourdieu, D. (1998a). A New Type of Flavor Precursors in Vitis vinifera L . cv . Sauvignon Blanc: S-Cysteine Conjugates. Journal of agricultural and food chemistry, 46, 5215-5219.
- Tominaga, T., Murat, M.-laure, & Dubourdieu, D. (1998b). Development of a Method for Analyzing the Volatile Thiols Involved in the Characteristic Aroma of Wines Made from Vitis vinifera L . Cv . Sauvignon Blanc. Journal of agricultural and food chemistry, 46, 1044-1048.

The preferred embodiments above described are obviously combinable. The following claims define further preferred embodiments of the present invention.

## Claims

1. *Saccharomyces cerevisiae* strain with the deposit under the number 13073, from 27^{th} March 2012, in CECT.

2. *S. cerevisiae* strain according to the previous claim, in its dried, lyophilized or paste forms.

3. Use of *S. cerevisiae* strain according to any of the preceding claims for food preparation by fermentation.

4. Vinification process comprising the use of the strain described in claim 1.

5. Vinification process according to claim 5 comprising the following steps:
adding to the must the strain described in claim 1 in appropriate amounts;
conducting the fermentation at a temperature between 16-28 °C until the fermentation ending.

6. Vinification process according to any one of claims 5-6 **characterized by** being applied to white and/or red grape must.

7. Vinification process according to the claim 6 **characterized by** must comprising grapes of at least one of the following white cultivars: Batoca, Loureiro, Alvarinho, Sauvignon Blanc, Chardonnay, Gewürztraminer, Riesling or mixtures thereof.

8. Vinification process according to the claim 7 **characterized by** must comprising grapes of at least one of the following red cultivars: Merlot, Cabernet Sauvignon, Syrah, Touriga Nacional, Vinhao or mixtures thereof.

9. Vinification process according to any of the claims 7-8 **characterized by** a white must fermentation temperature ranging between 25-28 °C.

10. Vinification process according to any of the claims 7-9, **characterized by** a red must fermentation temperature ranging between 25-28 ° C.

## Patentansprüche

1. *Saccharomyces cerevisiae* Stamm deponiert unter der Nummer CECT 13073, vom 27. März 2012.

2. *S. cerevisiae* Stamm gemäß dem vorherigen Anspruch, in seinen getrockneten, gefriergetrockneten oder pastösen Formen.

3. Verwendung eines *S. cerevisiae* Stammes gemäß einem der vorherigen Ansprüche zur Lebensmittelzubereitung durch Fermentation.

4. Verfahren zur Weinbereitung umfassend die Verwendung des in Anspruch 1 beschriebenen Stamms.

5. Verfahren zur Weinbereitung gemäß Anspruch 5 umfassend die folgenden Schritte:
Zugabe des unter Anspruch 1 beschriebenen Stamms in ausreichender Menge zum Most; Durchführen der Fermentation bei einer Temperatur zwischen 16-28 °C bis zum Ende der Fermentation.

6. Verfahren zur Weinbereitung gemäß einem der Ansprüche 5-6, **dadurch gekennzeichnet dass** es auf weißen und/oder roten Traubenmost angewendet ist.

7. Verfahren zur Weinbereitung gemäß Anspruch 6, das durch Most, der Trauben von mindestens einer der folgenden weißen Zuchtsorten umfasst, gekennzeichnet ist: Batoca, Loureiro, Alvarinho, Sauvignon Blanc, Chardonnay, Gewürztraminer, Riesling oder Mischungen davon.

8. Verfahren zur Weinbereitung gemäß Anspruch 7, das durch Most, der Trauben von mindestens einer der folgenden roten Zuchtsorten umfasst, gekennzeichnet ist: Merlot, Cabernet Sauvignon, Syrah, Touriga Nacional, Vinhão oder Mischungen davon.

9. Verfahren zur Weinbereitung gemäß einem der Ansprüche von 7-8, das durch die Fermentation von einem weißen Most bei einer Temperatur zwischen 25-28 °C gekennzeichnet ist.

10. Verfahren zur Weinbereitung gemäß einem der Ansprüche von 7-9, das durch die Fermentation von einem roten Most bei einer Temperatur zwischen 25-28 °C gekennzeichnet ist.

## Revendications

1. Souche de *Saccharomyces cerevisiae* déposée à la CECT sous le numéro 13073, du 27 mars 2012.

2. Souche de *S. cerevisiae* selon la revendication précédente sous sa forme séchée, liophilisée, ou de pâte.

3. Utilisation de la souche *S. cerevisiae* selon l'une quelconque des revendications précédentes pour la préparation d'aliments par fermentation.

4. Processus de vinification comprenant l'utilisation de la souche décrite dans la revendication 1.

5. Processus de vinification selon la revendication 5 comprenant les étapes suivantes :
ajouter au moût la souche décrite dans la revendication 1 en quantités appropriées ;
procéder à la fermentation à une température située entre 16°C et 28°C, jusqu'à ce que la fermentation soit conclue.

6. Processus de vinification selon l'une quelconque des revendications 5-6 **caractérisé en ce qu'**il s'applique au moût de raisin blanc et/ou rouge.

7. Processus de vinification selon la revendication 6 **caractérisé en ce que** le moût comprendre des raisins provenant au moins de l'un des cultivars blancs suivants : Batoca, Loureiro, Alvarinho, Sauvignon Blanc, Chardonnay, Gewürztraminer, Riesling, ou mélanges de ceux-ci.

8. Processus de vinification selon la revendication 7 **caractérisé en ce que** le moût comprendre des raisins provenant au moins de l'un des cultivars rouges suivants : Merlot, Cabernet Sauvignon, Syrah, Touriga Nacional, Vinhão, ou mélanges de ceux-ci.

9. Processus de vinification selon l'une quelconque des revendications 7-8, **caractérisé en ce que** la température de fermentation du moût blanc se situe entre 25°C et 28°C.

10. Processus de vinification selon l'une quelconque des revendications 7-9, **caractérisé en ce que** la température de fermentation du moût rouge se situe entre 25°C et 28°C.
